# EUROPEAN PATENT APPLICATION

(11) **EP 2 351 523 A1**
(43) Date of publication of application: **03.08.2011**
(21) Application number: 08877672.9
(22) Date of filing: 31.10.2008
(51) Int. Cl.: A61B 5/16, G06F 19/00

(54) **METHOD AND SYSTEM TO SAFELY GUIDE INTERVENTIONS IN PROCEDURES THE SUBSTRATE WHEREOF IS NEURONAL PLASTICITY**

(71) Applicant: Fundació Institut Guttmann, 08916 Badalona (Barcelona) (ES)
(72) Inventor: TORMOS MUÑOZ, José María, E-46135 Albalat dels Sorells (Valencia) (ES); GARCÍA RUDOLPH, Alejandro, 08034 Barcelona (ES); OPISSO SALLERAS, Eloy, 08301 Mataró (Barcelona) (ES); ROIG ROVIRA, María Teresa, 08029 Barcelona (Barcelona) (ES); GARCÍA MOLINA, Alberto, 08800 Vilanova i Ia Geltrú (Barcelona) (ES)
(74) Representative: Torner Lasalle, Elisabet
(86) International application number: PCT/ES2008/000677
(87) International publication number: WO 2010/049547

(57) **Abstract**

Method and system for safely guiding interventions in processes the substrate of which is the neuronal plasticity.

The method comprises generating a database with information regarding users in relation to interventions to be performed and to the user responses to the performance thereof, and analyzing it to generate candidate predictions from which final or optimum predictions are determined, said generation of candidate predictions and said subsequent determination of final predictions been carried out by means of corresponding steps of classification at different levels based on heuristic rules.
The system is provided for implementing the method proposed by the first aspect of the invention.
The method and the system are particularly applicable in processes such as those related to neurorehabilitation, neuroeducation/neurolearning or cognitive neurostimulation.

## Description

### Field of the Art

The present invention generally, and in a first aspect, relates to a method for safely guiding interventions in processes the substrate of which is the neuronal plasticity such as neurorehabilitation processes, neuroeducation/neurolearning processes or cognitive neurostimulation processes, by means of generating and using a database with information regarding a plurality of users, and particularly it relates to a method which comprises analyzing said database for generating candidate predictions, from which final or optimum predictions are determined, said generation of candidate predictions and said subsequent determination of final predictions been carried out by means of corresponding steps of classification at different levels.

A second aspect of the present invention relates to a system intended for implementing the method proposed by the first aspect of the invention.

### State of the Art

Different proposals regarding the supply or application to users or patients of interventions in processes the substrate of which is the neuronal plasticity such as those comprising cognitive tests or tasks, or of other type, and the subsequent evaluation of their responses, are known.

As described in "The Plastic Human Brain Cortex", Annual Reviews Neuroscience 2005.28:377-401 of Alvaro Pascual-Leone, Amir Amedi, Felipe Fregni and Lotfi B. Merabet, plasticity is an intrinsic property of the nervous system consisting in the capacity for modifying its structure from experience. This property allows it to learn, to acquire new skills, or even to recover from the alterations caused by a lesion. However, the changes do not necessarily result in a benefit; occasionally, these changes can generate the onset of diseases or be responsible for making the alterations derived from a lesion chronic. There is a challenge to sufficiently learn about neuronal plasticity for modulating it and thus achieving the best response of the behaviour for a specific patient. In order to carry out said modulation, as indicated in the aforementioned article, different types of interventions can be carried out such as those based on conduct modification, or those including different invasive or non-invasive cortical stimulation techniques.

Systems constructing databases with information associated with a plurality of users, to tasks or tests to be supplied, as well as to the responses of each user to the performance of the tasks which have been assigned to them are also known.

Some of said proposals describing methods and systems by means of which databases with more or less information regarding different users are created, as well as different ways of using the information of such databases for the purpose that the person responsible for selecting the intervention or interventions, such as a therapist, can evaluate the evolution of a user or a patient while executing one or more tasks, comparing his results with those of other users, or even selecting the tasks to be supplied to the user depending on the results of other users to whom said task has been assigned, are described below.

Patent US6964638 proposes a method for measuring the cognitive capacity of a user, presenting the user with a series of cognitive tests and, among other actions, performing a statistical analysis of the responses to said tests by using information which may include the responses to said tests of other presumably healthy users as reference. The specification of said patent advocates in that the proposal described therein combines the capacity of the statistical analysis with the capacity for collecting data from the responses to cognitive functions.

Patent US5711671 proposes a system including a host computer in connection with several computerized terminals of patients and of therapists. The host has access to a database including both tasks or treatments to be selected by a therapist to assign them to a patient, and the responses to the performance of said tasks by different patients. For one embodiment the host acts as a supervisor for the therapists. Other tasks that the host offers to the therapist are: registering patients on-line, prescribing (and updating) treatments, evaluating clinical progresses, as well as supplying reports. Said patent proposes that the therapist, once having seen the evolution of the patient, prescribes additional tasks or treatment processes to him. It also proposes that the host stores and combines the responses of several patients to enable performing a conductive search for rehabilitation processes.

In addition, patent US6280198 proposes a method for administering cognitive tests to a patient, remotely monitoring his responses to the tests and evaluating the evolution of his cognitive level based on said responses. Said patent proposes constructing a database which may include cognitive tests, of a reference type or not of a reference type, demographic information of the patient and his responses to the tests.

The specification of patent US6280198 describes including in the database information of responses to tests for a great number of persons and using this information for selecting proposed therapy programs.

US6280198 also describes performing, depending on its demographic characteristics or on other characteristics, different groups of patients having in common tendencies in their responses and including said groups in the database. Said groups have the purpose of extrapolating the responses of a patient belonging to a group to the rest of members of said group, in order, for example, to select a therapy program for a patient depending on the responses of other patients to similar therapy programs, as well as to evaluate the progress of the patient in comparison with other cases.

Patent US6632174 proposes a method for diagnosing and training cognitive skill of a user for the purpose of selecting one or more tasks to be performed by the user. It proposes storing in a database (local or remote) the responses and historical results of several users to enable performing a cross-validation of the results of a user against a considered acceptable criterion.

None of said documents teaches or even suggests the use of the aforementioned database beyond the direct use of said information stored in the database either for performing a statistic analysis, a conductive search, extrapolations or cross-validations of results for different patients.

### Description of the Invention

It is necessary to offer an alternative to the state of the art which allows actually extracting a great profit from the information stored in one of such databases, in relation to a plurality of users or patients, not for simply extracting it directly or for applying statistical criteria on it, but for using it as a raw data source to obtain a net information the classification of which, in different manners, provides a series of candidate predictions related to interventions to be performed or to which the user is to be subjected, based on which one or more final predictions are determined.

The present invention makes up the aforementioned alternative to the state of the art by means of providing, in a first aspect, a method the application of which provides the aforementioned use of the information of one of such databases to achieve the aforementioned objectives of selecting one or more final predictions which allow safely guiding interventions in processes the substrate of which is the neuronal plasticity.

To that end the present invention relates, in a first aspect, to a method for safely guiding interventions in processes the substrate of which is the neuronal plasticity, which comprises generating and using a database with information regarding a plurality of users at least in relation to interventions to be performed or to which to be subjected and to the user responses to the performance of said interventions.

Unlike the conventional proposals, the method proposed by the present invention characteristically comprises automatically performing the following steps:
a) generating two or more groups of candidate predictions related to possible interventions, performing two or more steps of classification based on at least heuristic rules on the information of said database considered as constituent of some basic training data,
b) generating from at least said two groups of candidate predictions a set of training data in meta-level, and
c) performing a meta-classification based on at least heuristic rules on said set of training data in meta-level, and
d) determining a group of optimum predictions based on the results of said step c), selecting one of said groups of candidate predictions obtained in step a), or combining them to one another.

For the purpose of improving the results to be obtained by means of applying the aforementioned step a), the proposed method comprises, for a preferred embodiment, validating, in step a), the results of said steps of classification from validation data common for validating the results of all the steps of classification, independently and separately from the basic training data, the candidate predictions being performed after said validation.

The method comprises, in order to obtain better results in said step c), generating in step b) the aforementioned set of training data in meta-level also from said validation data.

For one embodiment, said step a) comprises carrying out said steps of classification independently by means of using two or more classifiers, at least one for each step of classification, differentiated from one another at least in that each of them is based on the application of a respective set of heuristic rules different from that of the other classifier to obtain said two or more groups of candidate predictions different from one another.

Still in said embodiment, once the groups of candidate predictions have been generated, when said step d) comprises selecting one of the groups of candidate predictions, step d) also comprises selecting the classifier and heuristic rules used which have caused said optimum predictions.

For another embodiment of the method proposed by the first aspect of the invention, step a) comprises carrying out said two or more steps of classification by means of using a single classifier based on a single set of heuristic rules, said classifier being used twice or more, once for each step of classification with different input parameters every time to obtain said two or more groups of candidate predictions different from one another, after which for said case when step d) comprises selecting one of the groups of candidate predictions, this also comprises selecting the input parameters of the classifier which have caused said optimum predictions.

The two embodiments described in relation to the way for carrying out the steps of classification of step a) are alternative or complementary, which latter case contemplates performing steps of classification differentiated by using different classifiers, and other steps of classification differentiated in that, although they use one and the same classifier, the latter uses different input parameters every time. For said complementary case, the number of steps of classification is equal or more than three.

For the embodiment in which the steps of classification comprise using different classifiers, the method comprises using classifiers differentiated not only by the heuristic rules to be used but by other characteristics such as: the type of classifier, the operation mode, etc.

In addition, although the steps of classification and of meta-classification performed according to the proposed method are based on at least the application of heuristic rules, the proposed method comprises performing them by using other class of additional rules, such as deterministic rules.

For one embodiment, which will later be referred to as an off-line processing, the method comprises performing steps a) to d) prior to requesting or applying for a prediction in relation to an intervention for a determined user, in which case it comprises, for the purpose of carrying out said prediction, applying the classifier, together with its input parameters and heuristic rules selected after said step d) on data with information regarding said determined user to obtain at least the prediction in relation to an intervention to be performed.

For another alternative embodiment, or an on-line processing, the method comprises performing steps a) to d) after requesting the prediction in relation to an intervention for a determined user, data with information regarding said determined user being included in said database to be used in said step a) to finally obtain at least the prediction in relation to said intervention to be performed.

For the case in which said determined user is a user of the aforementioned plurality of users, the method comprises extracting the data with information regarding said determined user from the database for the purpose of using them in step a) as part of the aforementioned basic training data both for the off-line and on-line processing.

If, in contrast, said determined user is not a user of said plurality of users, i.e., if he is a new user, his data were therefore not incorporated in the database, the method comprises introducing the data with information regarding said determined user in said database both for the on-line and off-line processing, in the latter case the performance of steps a) to d) can be carried out once the data of the new user have already been incorporated in the database or prior to said introduction.

For the purpose of enriching the database and therefore increasing the precision in future predictions by applying the proposed method, the latter comprises:
- providing said intervention to said determined user;
- subjecting said determined user to said intervention; and
- acquiring and recording in said database data with information regarding the results of the determined user been subjected to said intervention and, if appropriate, other new data regarding said determined user for the purpose of updating said database.

The method comprises sequentially performing steps a) to d) again periodically or every time when new data is introduced in said database, the determined predictions, which will be more precise each time, thus being updated over time by the learning caused by executing steps a) to d) again and by updating the information stored in the database.

For an embodiment of the proposed method, the steps of classification for step a) and the step d) of meta-classification are carried out by means of using artificial neural networks, in which case the aforementioned input parameters are related to the parameters of an artificial neural network such as those referring to one or more of the following characteristics: network topology, activation function, end condition, learning mechanism, or to a combination thereof.

In an embodiment complementary to that described in the previous paragraph or independent thereof, the steps of classification for step a) and the step d) of meta-classification are carried out by means of using automatic inductive learning algorithms, carrying out in the step d) the selection of the inductive learning algorithm and/or of its input parameters which have caused the aforementioned optimum predictions.

For said case in which automatic inductive learning algorithms are used for the purpose of carrying out the above so-called off-line processing, the algorithms to be used are of greedy type (such is the case in which artificial neural networks are used).

In contrast, for the case above so-called on line processing, the algorithms to be used are of lazy type, such as those used in inductive methods like reasoning based on cases, the input parameters of which are one or more of the following parameters: indexing type (by sizes, by differences, by similarities, by explanations, etc.), storage type (dynamic memory model (Schank, Lolodner) or category and exemplar model (Porter, Bareiss)), recovery type (closest neighbors, decision trees, SQL type queries screen, etc.) and adaptation type (structural or derivational), or a combination thereof.

The proposed method comprises using any algorithm or strategy known in the field of meta-learning to carry out the different steps of classification described above.

The applications of the proposed method are whichever applications which include processes the substrate of which is the neuronal plasticity, such as those regarding neurorehabilitation, neuroeducation/neurolearning or cognitive neurostimulation, all of them representative of different embodiments of applying the method proposed by the invention.

In terms of the interventions safely guided by means of the proposed method, they comprise, for some embodiments, at least cognitive and/or functional tasks to be performed by the above so-called determined user or subject of said neurorehabilitation, neuroeducation/neurolearning or cognitive neurostimulation.

With regards to the information to be included in the database, the method proposed by the first aspect of the invention comprises including in said step for generating the database information regarding each user of said plurality of users in relation to personal variables and/or structural variables and/or functional variables and/or evolutionary variables which are defined in more detailed below.

With regards to said personal variables or the bio-psycho-social variables, these refer to all those variables making up the particular background of the life of the user or subject and of his lifestyle.

The present method distinguishes three types of personal variables:
- Biological variables: date of birth, age, sex, etc.
- Psychological variables: premorbid personality, attitudes of the person, coping styles, etc.
- Social variables: place of residence, education level, work status, socioeconomic status, marital status, stability, family support, etc.

The aforementioned structural variables comprise variables which allow defining whether alterations at a structural level exist, as well as for describing the involvement, if it exists, for each of the users, and they comprise one or more of the following variables:
- Primary and secondary (if any) diagnosis variables.
- Variables of etiology (e.g. TBI, stroke, neurodegenerative disease,...).
- Variables of lesions in neuroimaging.
- Variables of the severity of the lesion.
- Variables of time of evolution.

The aforementioned functional variables comprise information in relation to the cognitive aspects of the users assessed by means of a round of neuropsychological examination including one or more of the following variables:
- Attention variable which is a complex function formed by specifics sub-processes. The following are distinguished:
   1) Sustained attention: allows one to remain alert when facing stimuli for long time periods.
   2) Selective attention: capacity for selectively processing information inhibiting other not relevant information.
   3) Divided attention: capacity for performing two activities simultaneously.
- Language variable represents the capacity of human being for communicating to one another through signs, mainly through linguistic signs, the method distinguishes therein:
   - Production.
   - Comprehension.
   - Nomination
   - Reading
   - Grammar
   - Pragmatic
- Memory variable, defining the cognitive process which allows recording and reproducing information. Memory is not a single function, rather it can be subdivided based on different classifications like the following:
   - Time: long and short term memory.
   - Domain: declarative and implicit memory.
   - Type of information: verbal or non verbal.
   - Time phase: encoding, storing and retrieving.
- Executive functioning variable is the set of cognitive functions which allow controlling and regulating the conducts directed to an objective or goal, which are integrated by different cognitive capacities, and they include:
   - Planning.
   - Monitoring
   - Verifying
   - Inhibiting

Finally, in terms of the aforementioned evolutionary variables, these comprise information in relation to the success of each user been subjected to one or more interventions, said success being analyzed at least one of the following four levels:
- success at level of execution of the cognitive and/or functional task and of the suitability or adequacy of the task proposed for each specific profile of user;
- success at level of achievement of the immediate objective which is understood as an improvement in the cognitive function for which the cognitive and/or functional task has been selected;
- success at level of achievement of the generic objective which is understood as objectified improvements at other cognitive functions in addition to the target function; and
- success at level of achievement of long term objective which is understood as a reduction of the functional limitations for the development of daily activities in the case of a neurorehabilitation process, or which is understood as the achievement of a certain degree of neuroleaming in the case of a neuroeducation/neurolearning process, or which is understood as an improvement in the stimulated cognitive capacities in the case of cognitive neurostimulation.

The higher the number of said variables to be included in the database, the better the result to be obtained, i.e., the final predictions obtained will be more reliable. Therefore, for an embodiment, the method comprises including all the previously described variables in the database and using them as basic training data in step a).

For a preferred embodiment the method comprises starting said step a) after the prior selection, by the person responsible for selecting the intervention or interventions, of one or more interventions to be applied to a determined user.

With regards to the final predictions determined by means of applying the method proposed by the present invention, for a preferred embodiment these refer to the percentage of success or risk of applying an intervention to a determined user and the method comprises, for a variant of said embodiment, depicting said percentage of success or risk for said determined user by means of the previously described evolutionary variables, and incorporating the new values of the evolutionary variables for said determined user in the database.

If the so-called person responsible for selecting the intervention or interventions is a therapist selecting, for example, a task to be assigned to a patient (a selection that is carried out based on his knowledge on the subject), said therapist requires executing steps a) to d) of the proposed method to be guided in the intervention or task that has been previously selected.

Once steps a) to d) are executed, said guiding supplies him (for example visually through a screen) with a percentage of success or risk of assigning the selected task to the determined user, that percentage allowing the therapist to be guided in the sense of knowing if his selection is considered, by the system implementing the method, as a high or low risk selection, after which the therapist finally decides whether his selection of task is to be maintained or modified.

For the purpose of supplying, for example the therapist, with an additional source of guidance to the aforementioned percentage of success or risk, the method comprises performing a collaborative filtering by means of which integrating the explicit opinion of a plurality of therapists (or of persons responsible for other type of operation when is not the case of carrying out a therapy), by means of, for example, an individual weighting of the determined predictions.

A second aspect of the invention relates to a system for safely guiding interventions in processes the substrate of which is the neuronal plasticity which is suitable for applying the method proposed by the first aspect of the invention and which will be described in more detailed in a subsequent section.

### Brief Description of the Drawings

The foregoing and other advantages and features will be better understood from the following detailed description of several embodiments in reference to the attached drawings which must be interpreted in an illustrative and non-limiting manner, in which:
Fig. 1 shows a schematic diagram including the different elements taking part in the different steps of the method proposed by the first aspect of the invention for an embodiment; and
Fig. 2 is a schematic depiction of the system proposed by the second aspect of the invention for an embodiment.

### Detailed Description of Several Embodiments

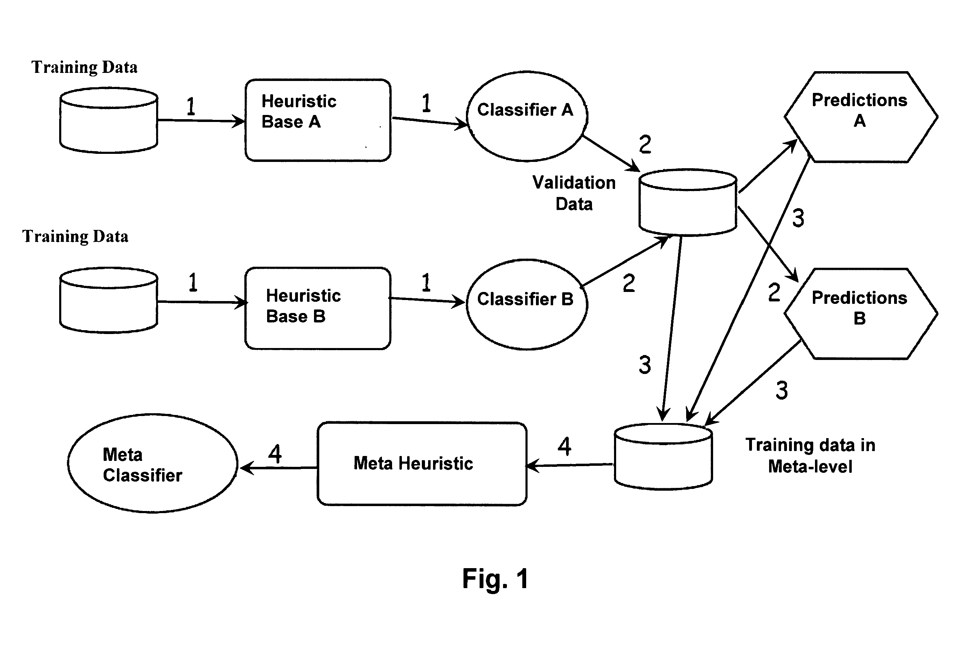

Firstly, referring to Fig. 1 in which, different elements or blocks by means of which steps a) to d) of the method proposed by the first aspect of the invention for an embodiment are implemented, have been depicted.

Specifically the diagram illustrated in Fig. 1 illustrates the previously described embodiment for which step a) comprises carrying out the steps of classification independently by means of using two classifiers indicated in Fig. 1 as "Classifier A" and "Classifier B", one for each step of classification, differentiated from one another in that each of them is based on the application of a respective set of heuristic rules different from that of the other classifier, indicated in Fig. 1 as "Heuristic Base A" and "Heuristic Base B" to obtain said two groups of candidate predictions different from one another indicated in Fig. 1 as "Predictions A" and "Predictions B", respectively.

Fig. 1 ultimately depicts a possible meta-learning scenario having the following steps:
1. Classifiers A and B are trained from a set of common training data, heuristic rules or heuristics base A and B, respectively, being applied on them. The sequence is indicated by arrows 1 in Fig. 1.
2. Candidate predictions A and B are generated from the classifiers A and B, respectively, learnt in a set of validation data independent and common for both classifiers. The sequence is indicated by arrows 2 in Fig. 1.
3. A set of training data in the meta-level is generated from the set of validation data and from the candidate predictions A and B generated by the classifiers A and B, respectively, in the set of validation data. The sequence is indicated by arrows 3 in Fig. 1.
4. The final classifier (Meta-classifier) is trained from the set of training data in Meta-level by means of Meta Heuristic using inductive learning in the meta level for integrating the different classifiers A and B, or for improving the performance of each of them independently for the purpose of determining the final or optimum predictions in the previously described step d).

The method proposed by the first aspect of the invention is carried out, for an embodiment, by means of the meta-learning scenario illustrated by Fig. 1, although for other embodiments, unlike the one illustrated, the scenario can be other scenario having greater or lesser complexity.

In terms of the strategies to be applied for implementing the Meta-classifier, these can be strategies of very diverse kinds such as the following: Voting, Weighted Voting or Arbitration for the purpose of obtaining the final prediction in step d) after receiving a request to be classified which, for an embodiment, consists of a task preassigned to a determined patient by a therapist.

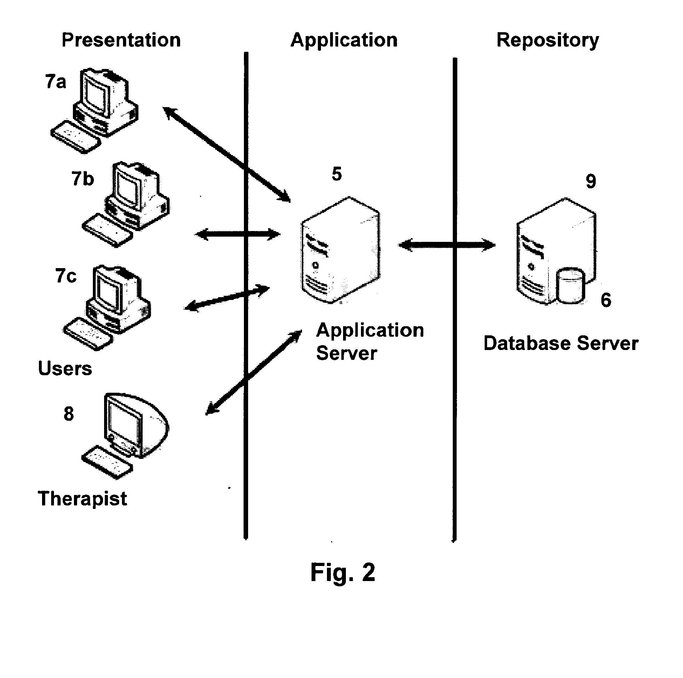

Fig. 2 illustrates an embodiment of the system proposed by the second aspect of the invention which is suitable for applying the method proposed by the first aspect of the invention, and it comprises:
- a central computer server 5 with access to a database 6 such as that described above for the method proposed by the first aspect of the invention,
- a plurality of computerized user computer terminals 7a, 7b, 7c in two-way communication with said central computer server 5 for receiving, each of them, information in relation to said interventions and for sending the result of the performing of said interventions to the central computer server 5, and
- a therapist computer terminal 8 in remote communication with said central computer server 5 for requesting the prediction of an intervention for a determined user, for receiving said requested prediction and for confirming that the information in relation to said intervention, in relation to which said prediction has been required, has been sent by the server to the terminal of said determined user 7a.

The central computer server 5 is provided for carrying out steps a) to d) of the method proposed by the first aspect of the invention.

An architecture of the proposed system divided into three layers has been depicted in said Fig. 2: one presentation layer including the different terminals 7a-7c and 8, one application layer including the aforementioned central server 5 referred to as application server and one repository layer, further including, other than the aforementioned database 6, a database server 9 through which the server 5 accesses the database 6.

An embodiment based on the architecture of the system illustrated in Fig. 2, which is also referred to as a platform, the operation of which is described below, is explained next.

Users can execute the tasks which have been programmed by the therapist regardless of their physical location through this platform, with the only requirements of having an Internet connection and the client software application installed in the computer or terminal of the user 7a-7c.

The platform server 5 provides a remote access web interface where the client software connects for the purpose of authenticating it, retrieving the information on the tasks to be performed in the current session and transmitting the generated results to the database server 9.

It therefore works based on an architecture structured in three independent layers that interact with one another:

### The presentation layer:

This layer joins all the aspects of the software that have to do with the interfaces and the interaction of the system with the users and therapists. The client software is installed in each of the computers, 7a-7c and 8, accessing the platform through the interface provided in this layer such that the therapists can provide criteria for the tasks to be executed by its users and these users executing them regardless of their physical location. This communication is performed by means of XML-RPC Web Services, which also operates through the HTTP or HTTPS protocol which a priori assures that the communications will not be blocked by routers or firewalls unless these have explicitly disabled the transmissions through ports 80 or 443. Since this protocol is executed over the TCP transport protocol, all the data sent will be received by the recipient.

### The Application Layer

Requests generated from the presentation layer by the client software are received and managed in this layer and the results are displayed. It interacts with the repository layer for requesting the storage or retrieval of data from the database server 9. In general terms, it is referred to as the layer where the logic of the method concentrates, i.e., the rules governing the behaviour at the operational level of the application, for the purpose of carrying out the method proposed by the first aspect of the invention for sending a final prediction to the therapist terminal 8, located in the presentation layer, after the therapist requested it and for sending the tasks assigned by the therapist to the user terminals 7a-7c.

### The repository layer:

This layer gathers all the aspects of the software that have to do with the management of the persistent data, managing them in a transparent manner at the application layer.

Some of the elements used based on the architecture structured in three layers illustrated by Fig. 2 are subsequently broken down. These are:
Design pattern: By taking into account this encapsulation of the system in these three independent levels or layers, the use of design pattern, Model-View-Controller (MVC), is proposed. This design pattern explicitly separates the data access and the logic of the method for presenting the data and the interactions with the users and therapists by means of introducing an intermediate component: the controller. For this purpose, the platform J2EE is used, where each of the three components of the design pattern will have the following functionality:
   Model: Any access to the databases 6 will use some of the functions provided by the classes of the Model. These classes are known as Data access Object (DAO) and they are applied from the classes known as Action. The classes which correspond to a representation of a table of a database are the Value Object (VO) detailed below.
   View: It corresponds to the web interface which is seen by the users and therapists and with which they must interact. It is implemented by using Java Server Pages (JSP) with HTML and CSS code.
   Controller: It is a Servlet which receives the requests from View and which directs them to the corresponding Model.
Every web application in the Apache Tomcat server, which is the one used in the system for an embodiment, contains two non-public directories of information in relation to the execution of the web. These directories are:
   META-INF: It contains the file manifest.mf with generic information on the application and the file context.xml which defines the context with resources used by the web, such as for example the access to database 6.
   WEB-INF: It contains the compiled classes, libraries and the file web.xml defining the structure of the application with the existing servlets, redirections and maps. It also contains some so-called property files which are the following:
      - actions.properties: indicates for each entity which is the file with the view (JSP) and which is the file with the action (Action).
      - params.properties: defines the general parameters of the application.
   The process followed by the system serving as a webpage is described below:
      1. A request is generated from a URL direction with the format protocolo://server:puerto/peticion.do. Since the directions ended in .do are mapped to the controller, as has been defined in the file web.xml, the latter will receive the request.
      2. The controller will pass the captured request to the next action, since it is loaded with the file action.properties, the action that must be executed is known.
      3. The action corresponding to the request receives the data from the former and provides the corresponding queries to the database 6 through the DAO corresponding to the entities from which data must be retrieved, inserted, deleted or updated.
      4. When the action ends, the controller calls the view corresponding to the request.
      5. The view (JSP) receives the data returned by the action, encapsulated in the variable of response type. The view contains Java code which processes the received data (collecting VO, etc.), compiles them in execution time and sends the resulting HTML code to the web navigator.

The Model of the design pattern is therefore made up of Entities which are J2EE components representing the data stored in the database. Each entity corresponds to one of the following three objects:
- Data access Object: implements the class containing all the methods for providing queries to the database; these queries allow retrieving, inserting, deleting and updating data.
- Value Object: these are objects used for transferring information between processes and have no other behavior than to store and retrieve their own data.
- Primary Key: This object is a class which stores the primary key of the entity. This type of primary key is defined in this class as the one which allows detaching it in all the platform.

Some of the entities implemented in the platform are presented below:
- User: Manages all data related to the users of the application regardless of the role of each of them (users, therapists,...).
- Language: Serves to manage the languages (Spanish, Catalan,..) contemplated by the platform and allows aggregating new ones.
- Function: It is used to save the information relating to supported functions (cognitive,...) and sub-functions.
- Task: It contains the information in relation to the tasks proposed by the therapists to the users.
- Parameters: It contains the information of the input and output parameters of a given task.
- Session: It contains the data related to sessions of tasks

The planning of tasks and the query of results have the peculiarity that an action on some of the elements of the page does not cause the page to be completely refreshed but rather only those parts containing new information are refreshed. This is achieved by using a group of interrelated web development techniques known as AJAX (Asynchronous JavaScript and XML). This technology allows performing asynchronous calls in JavaScript to a web server by using the object XMLHttpRequest, the response thereof is also processed in an asynchronous manner for dynamically changing the outlook of the webpage. This provides a greater interactivity, functionality, efficiency and ease of use of the webpages.

On the application server 5 side three servlets, which are those that receive these asynchronous requests for the planning of tasks section, have been created. These servlets are:
BlockServlet: It manages the blocks of tasks and the tasks within them, it also allows changing day in the calendar, loading the plan on the selected day.
SchedulerServlet: It navigates the tree of tasks generated by grouping them into functions and sub-functions.

With regard to the query of results, the following servlet is used:
ResultServlet: it allows navigation by a calendar, loading the information in relation to the results of the selected session for each selected day.

On the client side, i.e., the navigator, the files ajax.js for planning and query of results have been created. These files contain all the functions which are used for communicating in an asynchronous manner with the server 5. In order to avoid the implementation of the HTTP calls, a free code library known as SACK, which provides an API which facilitates calling the server as well as obtaining the response, is used.

Regarding to the responses received by the server 5, they can be in plain text format or in the XML format. In the first case, it is read directly, if it is of XML format the XML DOM functions of JavaScript are used to read the received data. In both cases, in order to dynamically modify the display of the webpage the JavaScript functions known as HTML DOM are used.

As mentioned above, the system allows that the users to not be physically located in any particular location (hospital, care centre, etc.) to perform the tasks assigned by the therapists after they have been conveniently guided for measuring the final prediction determined according to step d) of the method proposed by the first aspect of the invention. The technology selected for supporting this performance are the XML-RPC web services due to its simplicity, minimalism and ease of use. The data are sent in XML format and the conversions between remote calls and XML are performed by the libraries in a transparent manner to the programmer. It further allows abstracting the web application and the client software from the programming language used.

The extensive and specific description of the previous embodiment has demonstrated that the system proposed by the second aspect of the invention has been implemented in practice to demonstrate the suitability thereof and of the method proposed by the first aspect of the invention applied by the system.

A person skilled in the art could introduce changes and modifications in the described embodiments without departing from the scope of the invention as defined in the attached claims.

## Claims

1. A method for safely guiding interventions in processes the substrate of which is the neuronal plasticity, of the type which comprises generating and using a database with information regarding a plurality of users at least in relation to interventions to be performed or to which to be subjected and to the users responses to the performance of said interventions, said method being **characterized in that** it comprises automatically performing the following steps:
a) generating at least two groups of candidate predictions related to possible interventions, performing at least two steps of classification based on at least heuristic rules on the information of said database considered as constituent of some basic training data,
b) generating from at least said two groups of candidate predictions a set of training data in meta-level, and
c) performing a meta-classification based on at least heuristic rules on said set of training data in meta-level, and
d) determining a group of optimum predictions based on the results of said step c), selecting one of said groups of candidate predictions obtained in step a), or combining them to one another.

2. The method according to claim 1, **characterized in that** said step a) comprises carrying out said at least two steps of classification independently by means of using two respective classifiers differentiated from one another at least **in that** each of them is based on applying a respective set of heuristic rules different from that of the other classifier to obtain said at least two groups of candidate predictions which are different from one another.

3. The method according to claim 2, **characterized in that** when said step d) comprises selecting one of said groups of candidate predictions, step d) also comprises selecting the classifier and heuristic rules used which have caused said optimum predictions.

4. The method according to claim 1, **characterized in that** said step a) comprises carrying out said at least two steps of classification by means of using a single classifier based on a single set of heuristic rules, said classifier being used at least twice, once for each step of classification with different input parameters every time.

5. The method according to claim 4, **characterized in that** when said step d) comprises selecting one of said groups of candidate predictions, step d) also comprises selecting the input parameters of said classifier which have caused said optimum predictions.

6. The method according to claim 3 or 5, **characterized in that** it comprises performing said steps a) to d) prior to requesting or applying for a prediction in relation to an intervention for a determined user, and **in that** it comprises, for the purpose of carrying out said prediction, applying the classifier together with its input parameters and heuristic rules selected after said step d) on data with information regarding said determined user to obtain at least the prediction in relation to an intervention to be performed.

7. The method according to claim 3 or 5, **characterized in that** it comprises performing said steps a) to d) after requesting the prediction in relation to an intervention for a determined user, data with information regarding said determined user being included in said database to be used in said step a) to finally obtain at least the prediction in relation to said intervention to be performed.

8. The method according to claim 6 or 7, **characterized in that** if said determined user is a user of said plurality of users, the method comprises extracting said data with information regarding said determined user from said database.

9. The method according to claim 6 or 7, **characterized in that** if said determined user is not a user of said plurality of users, the method comprises introducing said data with information regarding said determined user in said database.

10. The method according to claim 8 or 9, **characterized in that** it comprises:
- providing said intervention to said determined user;
- subjecting said determined user to said intervention; and
- acquiring and recording in said database data with information regarding the results of the determined user been subjected to said intervention and, if appropriate, other new data regarding said determined user for the purpose of updating said database

11. The method according to claim 10, **characterized in that** it comprises sequentially performing said steps a) to d) again periodically or every time new data is introduced in said database.

12. The method according to any of the previous claims, **characterized in that** said step a) comprises validating the results of said steps of classification from validation data common for validating the results of all the steps of classification, the candidate predictions being performed after said validation.

13. The method according to claim 12, **characterized in that** said step b) comprises generating said set of training data in meta-level also from said validation data.

14. The method according to any one of the previous claims when it depends on claim 4 or 5, **characterized in that** said steps of classification of said step a) and said step d) of meta-classification are carried out by means of using artificial neural networks, said input parameters being at least related to one of the following characteristics of an artificial neural network: network topology, activation function, end condition, learning mechanism, or to a combination thereof.

15. The method according to any of the previous claims, **characterized in that** said steps of classification of said step a) and said step d) of meta-classification are carried out by means of using automatic inductive learning algorithms, carrying out in said step d) the selection of the inductive learning algorithm and/or of its input parameters which have caused the aforementioned optimum predictions.

16. The method according to claim 15 when it depends on claim 6, **characterized in that** said performing of steps a) to d) prior to requesting or applying for a prediction in relation to an intervention for a determined user forms part of the execution of greedy automatic inductive learning algorithms.

17. The method according to claim 15 when it depends on claim 7, **characterized in that** said performing of steps a) to d) after requesting or applying for a prediction in relation to an intervention for a determined user forms part of the execution of lazy type automatic inductive learning algorithms.

18. The method according to any of the previous claims, **characterized in that** said step for generating said database comprises including information regarding each user of said plurality of users in relation to personal variables and/or structural variables and/or functional variables and/or evolutionary variables.

19. The method according to claim 18, **characterized in that** said process the substrate of which is the neuronal plasticity is a neurorehabilitation process.

20. The method according to claim 18, **characterized in that** said process the substrate of which is the neuronal plasticity is a neuroeducation/neurolearning process.

21. The method according to claim 18, **characterized in that** said process the substrate of which is the neuronal plasticity is a cognitive neurostimulation process.

22. The method according to claim 19, 20 or 21, **characterized in that** said interventions comprise at least cognitive and/or functional tasks to be performed by said determined user or subject of said neurorehabilitation, of said neuroeducation/neurolearning or of said cognitive neurostimulation.

23. The method according to any one of claims 18 to 22, **characterized in that** said personal variables comprise biological variables and/or psychological variables and/or social variables.

24. The method according to any one of claims 18 to 23, **characterized in that** said structural variables comprise variables which allow defining whether alterations at a structural level exist, as well as describing the involvement, if it exists, for each of the users, and they comprise primary and secondary diagnosis variables, if appropriate, and/or variables of etiology and/or variables of lesions in neuroimaging and/or variables of the severity of the lesion and/or variables of time of evolution.

25. The method according to any one of claims 18 to 24, **characterized in that** said functional variables comprise information in relation to the cognitive aspects of the users assessed by means of a round of neuropsychological examination, and they comprise attention variables and/or language variables and/or memory variables and/or executive functioning variables.

26. The method according to claim 22, **characterized in that** said evolutionary variables comprise information in relation to the success of each user been subjected to one or more interventions, said success being analyzed at at least one of the following four levels:
- success at level of execution of the cognitive and/or functional task and of the suitability or adequacy of the task proposed for each specific profile of user;
- success at level of achievement of the immediate objective which is understood as an improvement in the cognitive function for which the cognitive and/or functional task has been selected;
- success at level of achievement of the generic objective which is understood as objectified improvements at other cognitive functions in addition to the target function; and
- success at level of achievement of the long term objective which is understood as a reduction of the functional limitations for the development of daily activities in the case of a neurorehabilitation process, or which is understood as the achievement of a certain degree of neurolearning in the case of a neuroeducation/neurolearning process, or which is understood as an improvement in the stimulated cognitive capacities in the case of cognitive neurostimulation.

27. The method according to any of the previous claims, **characterized in that** it comprises starting said step a) after the prior selection by the person responsible for selecting the intervention or interventions of at least one intervention to be applied to a determined user.

28. The method according to claim 27, **characterized in that** said predictions refer to the percentage of success or risk of applying an intervention to a determined user.

29. The method according to claim 28 when it depends on claim 26, **characterized in that** it comprises depicting said percentage of success or risk for said determined user by means of said evolutionary variables and incorporating the new values of the evolutionary variables for said determined user in the database.

30. A system for safely guiding interventions in processes the substrate of which is the neuronal plasticity, comprising:
- a central computer server (5) with access to a database (6) with information regarding a plurality of users at least in relation to interventions to which to be subjected or to be performed and to the user responses to the performance of said interventions,
- a plurality of computerized user computer terminals (7a, 7b, 7c) in two-way communication with said central computer server (5) for receiving, each of them, information in relation to said interventions and for sending the result of performing said interventions to the central computer server (5), and
- at least one therapist computer terminal (8) in remote communication with said central computer server (5) for requesting the prediction in relation to at least one intervention for a determined user, for receiving said requested prediction and for confirming that information in relation to said intervention, in relation to which said prediction has been required, has been sent by the server to the terminal of said determined user (7a),
said system being suitable for applying the method according to any one of claims 1 to 29.

31. The system according to claim 30, **characterized in that** said central computer server (5) is provided for carrying out said steps a) to d).
